(19) Europäisches Patentamt
European Patent Office
Office européen des brevets

(11) **EP 3 725 221 A1**

(12) **EUROPEAN PATENT APPLICATION**

(43) Date of publication:
**21.10.2020 Bulletin 2020/43**

(51) Int Cl.:
*A61B 5/0496* (2006.01)    *A61B 5/00* (2006.01)
*A61B 5/16* (2006.01)    *A61B 3/113* (2006.01)

(21) Application number: **19170306.5**

(22) Date of filing: **18.04.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Stichting IMEC Nederland
5656 AE Eindhoven (NL)**

(72) Inventors:
• **MERONI, Alessio
3001 Leuven (BE)**

• **PRADHAPAN, Paruthi
3001 Leuven (BE)**
• **VAN DER HEIJDEN, Patrick
3001 Leuven (BE)**
• **SHAHRIARI, Navid
3001 Leuven (BE)**
• **BAX-WITTEVEEN, Jolanda
3001 Leuven (BE)**

(74) Representative: **Patent Department IMEC
IMEC vzw
Patent Department
Kapeldreef 75
3001 Leuven (BE)**

(54) **SYSTEM, DEVICE AND METHOD FOR EYE ACTIVITY MONITORING**

(57)    An electronic system (1) for monitoring eye activity of a subject, comprising: a plurality of EOG electrodes (103,A,B,C,D,REF); electrode circuitry (104) configured for measuring an adapt EOG signals received from the plurality of electrodes (103,A,B,C,D,REF); and a data processing unit (106) configured for performing EOG signal processing and eye activity determination; wherein the data processing unit (106) is configured for performing eye activity determination based on at least a first diagonal eye biosignal vector (Diagonal 1) derived from the biosignals measured between at least a first electrode pair (B,C) that, in operation, is located on a first diagonal plane (D1) around a subject's eye. A wearable device (100,200) and a method for monitoring eye activity of a subject.

**Figure 6**

## Description

### Technical Field

The present description relates generally to electronic systems for monitoring eye activity of a subject, and more specifically, to electronic systems for monitoring eye activity of a subject using electrooculogram (EOG) biosignals.

### Background

Electrooculography is a methodology for eye-activity monitoring based on the measurement of the electric potential variation on the skin around the eyes due to the rotation of the eyes themselves. This methodology allows measurement of eye-movements and is particularly effective for rapid eye movements (saccades) and blinks.

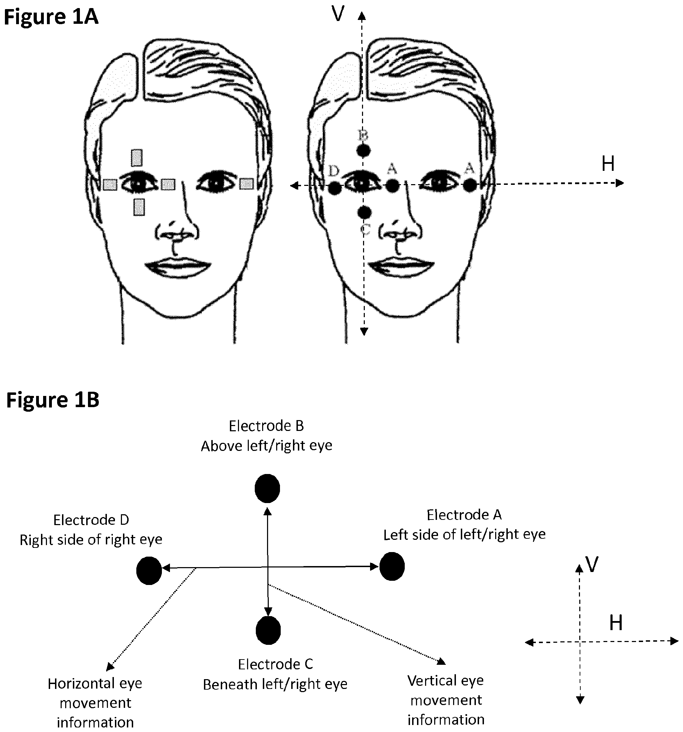

Traditional EOG measurement consist of the placement of at least four measurement electrodes. In **Figure 1A** a commonly used electrode configuration for tracking eye activity is shown. This electrode position allows for a measure of the horizontal eye movements through a first pair of electrodes **A&D**, and vertical eye movements and blinks through a second pair of electrodes **B&C**. Electrode **A** can be placed on two locations: as close as possible horizontally next to the eye where electrode **D** is placed, or on the other side of the face as where electrode **D** is placed. **Figure 1B** shows a vector plot of a known EOG electrode configuration setup corresponding to **Figure 1A**. The electrodes **A,B,D,C** are placed such that the EOG signal activity vectors are orthogonal to each other, thus minimizing cross-talk between the horizontal and vertical eye activity channels.

### Summary

The present description proposes a new and improved electronic system, device and method for eye activity monitoring using EOG biosignals information.

The invention is defined by the claims. An electronic system for monitoring eye activity of a subject is defined in claim 1. A wearable device for monitoring eye activity of a subject is defined in claim 5 and a method for monitoring eye activity of a subject is defined in claim 8.

According to an example embodiment, the electronic system for monitoring eye activity of a subject according to the present description allows for a new EOG electrode configuration that can be implemented in a more convenient form factor.

Advantageously, according to an example embodiment, the electronic system for monitoring eye activity of a subject according to the present description, allows for placing the EOG electrodes in locations around the eye that can be easily integrated in standard glasses, smart-glasses and/or smart-goggles.

According to an example embodiment, the electronic system for monitoring eye activity of a subject according to the present description advantageously provides an improved signal-to-noise ratio for signals deriving from the EOG electrodes. The EOG biosignals present a larger in amplitude with respect to prior art electrode layouts and the SNR is improved, especially when the signals are combined to extract vertical and lateral/horizontal eye movements.

According to an example embodiment, the electrode location advantageously allows to minimize the overlap with face muscles in order to minimize the impact of artifacts due to facial expressions.

### Brief description of the drawings

The above and other aspects of the electronic system, device and method according to the present description will be shown and explained with reference to the non-restrictive example embodiments described hereinafter.

**Figure 1A** shows a prior art EOG electrode configuration for eye activity measurements.

**Figure 1B** shows an eye activity vector plot for a prior art EOG electrode configuration according to Figure 1A.

**Figure 2** illustrates an EOG electrode location configuration in the face of a subject according to an example embodiment of the present description.

**Figure 3A** is an example illustration of an eye activity vector plot for an EOG electrode configuration as in **figure 2**.

**Figure 3B** is an example illustration of a vector combination of diagonals to derive the horizontal and vertical eye activity according to an EOG electrode configuration as in **figure 2**.

**Figure 4A** is an example flow chart for horizontal and vertical eye-movements determination from two diagonal eye EOG signals.

**Figure 4B** is an example flow chart illustrating a method for blink determination and eye spectral/statistical analysis based on a single diagonal eye EOG signal.

**Figure 5** illustrates the function of a data processing unit for eye activity monitoring according to an example embodiment.

**Figure 6** shows a system for eye activity monitoring according to a first example embodiment.

**Figure 7** shows a system for eye activity monitoring according to a second example embodiment.

**Figure 8** shows an electrode circuitry module from a system for eye activity monitoring according to an example embodiment.

**Figure 9** shows a perspective view of a device for eye activity monitoring in the form factor of a pair of glasses according to an example embodiment.

**Figure 10** shows a top view of the device for eye activity monitoring of **figure 9.**

**Figure 11** illustrates a method for measuring EOG signals in a system for eye activity monitoring according to a first example embodiment.

**Figure 12** illustrates a method for measuring EOG signals in a system for eye activity monitoring according to a second example embodiment.

**Figure 13** shows a graph of the horizontal eye activity information derived from diagonal electrode pairs according to an example embodiment compared to a prior art conventional electrode configuration.

## Detailed description

In the following, in the description of example embodiments, various features may be grouped together in a single embodiment, figure, or description thereof for the purpose of streamlining the disclosure and aiding in the understanding of one or more of the various inventive aspects. This is however not to be interpreted as the invention requiring more features than the ones expressly recited in the main claim. Furthermore, combinations of features of different embodiments are meant to be within the scope of the invention, as would be clearly understood by those skilled in the art. Additionally, in other instances, well-known methods, structures and techniques have not been shown in detail in order not to obscure the conciseness of the description.

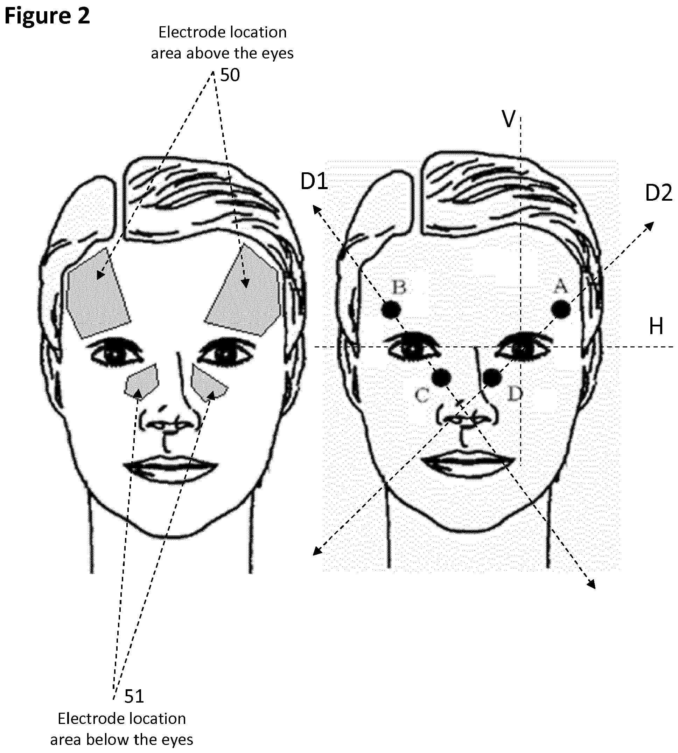

**Figure 2** shows an EOG electrode location configuration in the face of a subject according to an example system according to the present description. According to an example embodiment, two electrodes **C,D** are placed on the nose or in proximity of the nose below the eyes and another two electrodes **A,B** are placed in the facial area between temporal and frontalis muscles. According to an example embodiment, two EOG electrodes **C,D** may be located in the region touching the nose of the subject **51** and two additional electrodes **A,B** may be touching or being placed in the facial area of the subject between temporal and frontalis muscles **50**. According to an example embodiment, a first electrode **A** is placed at the left temple area, a second electrode **B** is placed at the right temple area; a third electrode **C** is placed at the right nose area, and a fourth electrode **D** is placed at the left nose area. According to an example embodiment, the electrodes form diagonal pairs, that is, a first pair of electrodes **B,C** located on a first diagonal plane **D1** around the right eye and a second pair of electrodes **A,D** located on a second diagonal plane **D2** around the left eye. The diagonal planes **D1,D2** in which the electrodes are located, are neither parallel nor perpendicular to the horizontal plane **H** defined by of the eyes of the subject. According to an example, the diagonal planes **D1,D2** are substantially at a 45 or 135 degree angle with respect to the horizontal plane **H.** The shaded or grey areas **50,51** in the figure represent the area of the subject's face that are well-suited for electrode positioning. These shaded areas are close to the eyes, which is needed for detecting the eye activity.

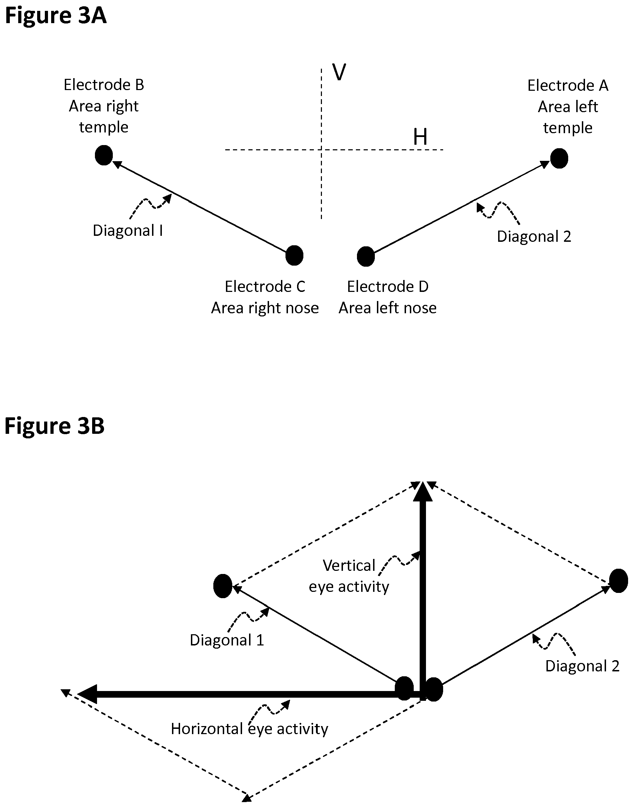

According to an example, the system is configured to measure the electrical eye activity between the first pair of electrodes **B,C** and between the second pair of electrodes **A,D**. These measurements define a first diagonal eye biosignal vector (**Diagonal 1** in **Figure 3A**) and a second diagonal eye biosignal vector (**Diagonal 2** in **Figure 3A**). Advantageously, the location of the electrodes **A,B,C,D** may be optimized to minimize the amount of noise originating from the facial muscles which could interfere with the eye activity measurement, and to minimize the amount of skin movements (which cause motion artifacts) at those electrode locations. According to an example embodiment, the electrodes **A,B,C,D** are positioned such that muscle and movement artifacts are minimized, while the signal-of-interest is maximized. Furthermore, it is advantageous that the electrode position is comfortable for the user and adaptable to a widely accepted wearable form factor such as glasses.

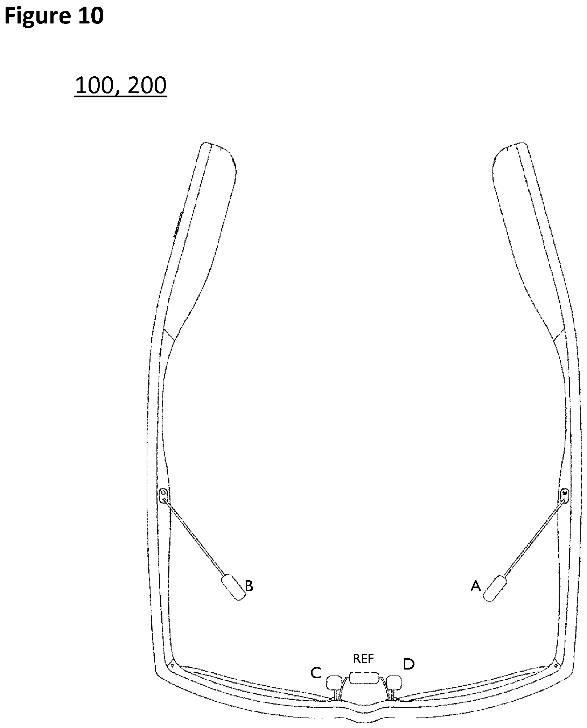

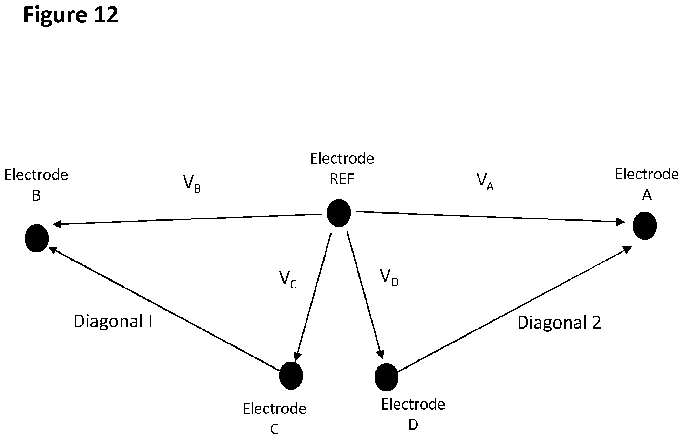

A four-electrode configuration as shown in **figures 2, 3, 11** allows detection of horizontal and vertical eye movements and blinks. According to another embodiment, a five-electrode configuration (comprising a reference electrode, as shown in **figures 10, 12**) may be also used for the same purpose. According to another embodiment, a two-electrode configuration (for example one single pair of electrodes **B,C** or **A,D** in **figure 2**) may be also used for blink detection and statistical and spectral analysis of eye-activity.

**Figure 3A** shows an eye activity signal vector plot for an EOG electrode configuration and measurement according to **figure 2**. According to an example embodiment, a first diagonal vector **Diagonal 1** and second diagonal vector **Diagonal 2** are measured and determined. The diagonal vectors are neither located in a horizontal plane **H** nor in a plane orthogonal to that horizontal plane. A horizontal and a vertical eye activity information, including blinks, may be mathematically derived by combining both diagonal vectors **Diagonal 1**, **Diagonal 2,** as shown in **figure 3B**. According to another embodiment, blink information and eye-activity may be also derived from a single diagonal vector **Diagonal 1** or **Diagonal 2**. According to an example embodiment, the horizontal and vertical vectors can be derived from the diagonal EOG signal vectors **Diagonal 1** and **Diagonal 2** as follows:

$$\text{EOG Horizontal} = \text{Diagonal 1} - \text{Diagonal 2}$$

$$\text{EOG Vertical} = \text{Diagonal 1} + \text{Diagonal 2}$$

$$\text{EOG Blink} = f\,(\text{Diagonal 1}) \text{ or } f\,(\text{Diagonal 2})$$

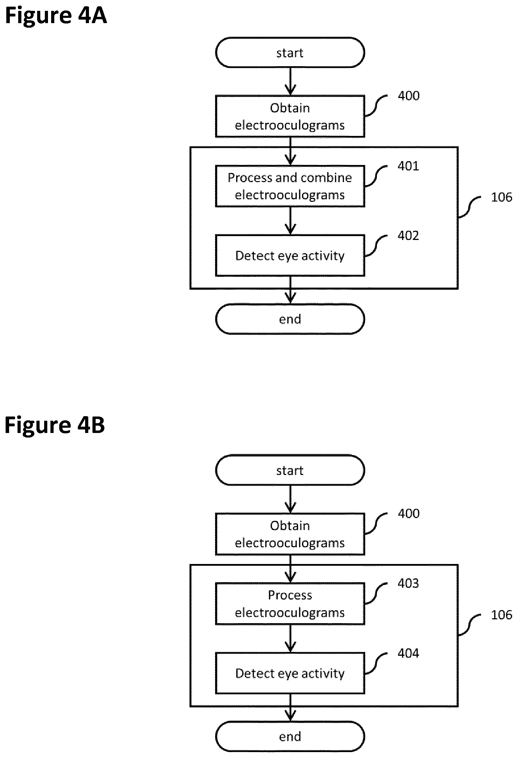

**Figure 4A** is an example flow chart for horizontal and vertical eye-movements determination from two diagonal eye EOG signals in a system with electrodes located as in **figure 2**. According to an example embodiment, first the electrical activity of the eyes is measured **400** by the four EOG electrodes **A,B,C,D** in a configuration as shown in **figure 2**. The signals coming from the four electrodes are processed and combined **401** as in **figures 3A** and **3B**, and then used to detect eye-activity **402**.

According to another embodiment, shown in **figure 4B**, the electrical activity of an eye is measured by two diagonal EOG electrodes **400**, for example a single pair of electrodes (**B,C** or **A,D**). The signals coming from the two electrodes are then processed **403** and used to detect eye-activity **404**. The system for eye activity monitoring according to an embodiment, comprises a data processing unit **106** that is configured for performing EOG signal processing, EOG signal combination and eye activity determination.

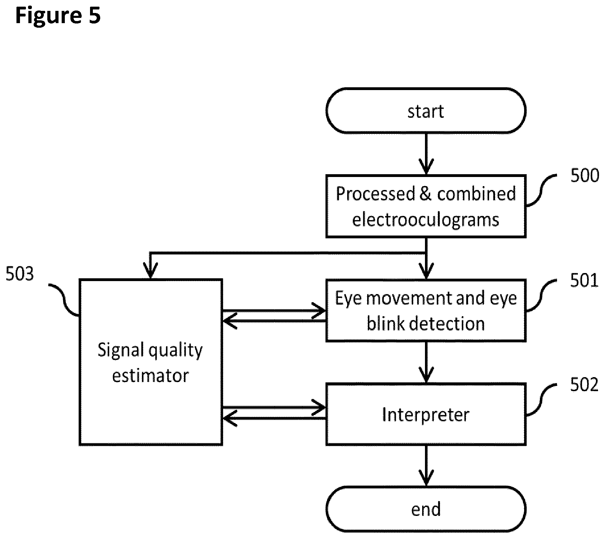

**Figure 5** illustrates the function of a data processing unit **106** for eye activity monitoring according to an example embodiment. In a first step, a first processing module **500** receives EOG biosignal data from the electrodes and associated electronics and, depending on the number of electrodes measured, it processes (in case of a single electrode pair and a single diagonal vector calculation) and/or combines the signals (in case of two electrode pairs and two diagonal vector calculations). An interpreter module **502** interprets the output data from an eye movement and eye blink detection module **501**, by combining or discarding or passing-through the detected eye movements and eye blinks. According to an example embodiment, the interpreter module **502** is configurable for different application requirements. A signal quality estimator module **503** receives the EOG signals from the first processing module **500** and estimates the quality of the signal. The signal quality estimator module **503** communicates with the eye movement and eye blink detection module **501** and the interpreter module **502** in order to adapt internal settings. The interpreter module **502** may determine and provide the following information: eye movements information (e.g. type, direction, magnitude, timing, etc.); eye blinks information (e.g. timing, voluntary/involuntary, etc.); and/or eye activity spectral and statistical analysis.

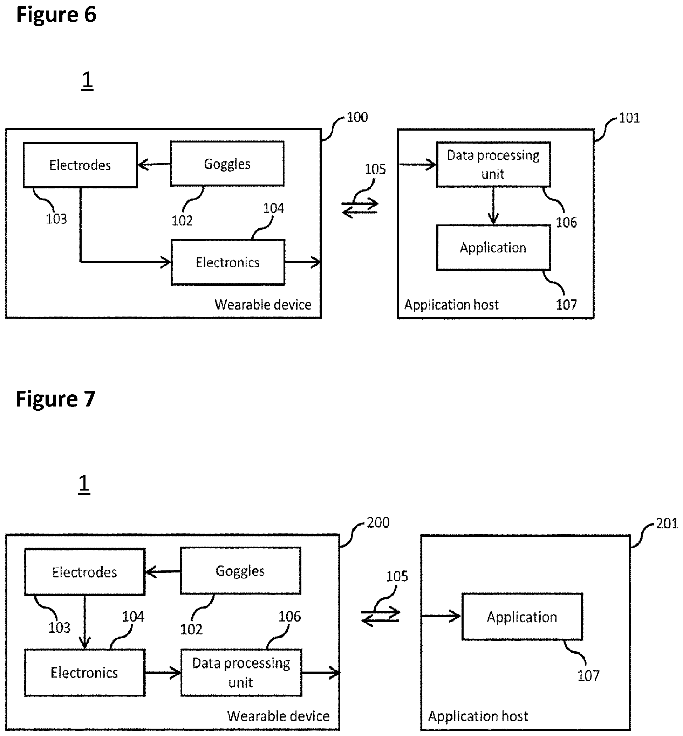

**Figure 6** shows a system **1** for monitoring eye activity of a subject according to a first example embodiment, comprising a plurality of EOG electrodes **103** and associated electrode circuitry **104** integrated in a wearable device **100**. The wearable device **100** may comprise glasses, googles, eye lenses or imaging units **102**. The wearable device is worn by the subject on the face and can have the form factor of a pair of glasses with integrated electrodes and associated electrode circuitry. Other form-factors are possible, like augmented (AR), mixed or virtual reality (VR) glasses or display devices. The wearable device **100** is communicatively coupled, via a wired or wireless connection unit **105,** to an application host unit **101**. According to an example embodiment, the application host unit **101** comprises a data processing unit **106** as, for example, described in **figure 5**. The data processing unit **106** is configured then to provide eye activity information to an application module **107**.

**Figure 7** shows a system **1** for monitoring eye activity of a subject according to a second example embodiment, comprising a plurality of EOG electrodes **103,** associated electrode circuitry **104** and a data processing unit **106** (as, for example, described in **figure 5**), integrated in a wearable device **200**. The wearable device **200** may comprise glasses, googles, eye lenses or imaging units **102**. The wearable device is worn by the user on the face and can have the form factor of a pair of glasses with integrated electrodes and associated electrode circuitry. Other form-factors are possible like augmented, mixed or virtual reality glasses or display devices. The wearable device **200** is communicatively coupled, via a wired or wireless connection unit **105**, to an application host unit **201**. The application host unit **201** comprises an application module **107** that receives eye activity information from the data processing unit **106**.

The data processing unit **106** can be executed on an external computing device (as in **figure 6**) or can be embedded on the wearable device (as in **figure 7**), for example a pair of glasses. According to an embodiment, the wearable device **100, 200** may be implemented and given the form of a pair of glasses or goggles, which will be the wearable structure that holds the electrodes and the electronics in place. The application host **101,201** may be a physical device or a cloud service.

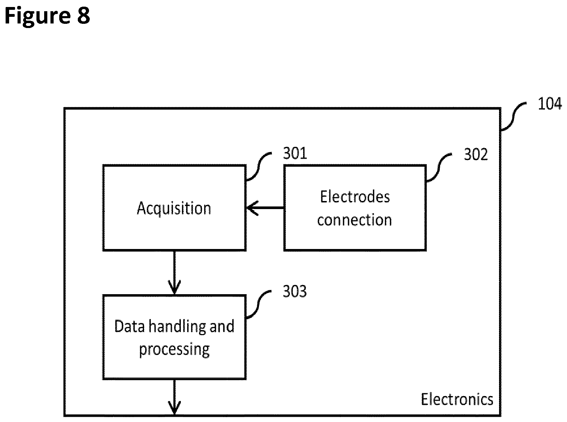

**Figure 8** shows an example electrode circuitry module **104** from a system for eye activity monitoring as shown in **figures 6, 7**, comprising an EOG signal acquisition electronic unit **301**, an electrode connection unit **302** and an EOG signal handling and processing unit **303**. The electrode circuitry module **104** may be integrated in the wearable device **100, 200**. Eye activity signals are captured by EOG signal acquisition electronic unit **301**. The EOG signal handling and processing **303** treats the signals and data and passes it to either the data processing unit **106** or to the connection unit **105**.

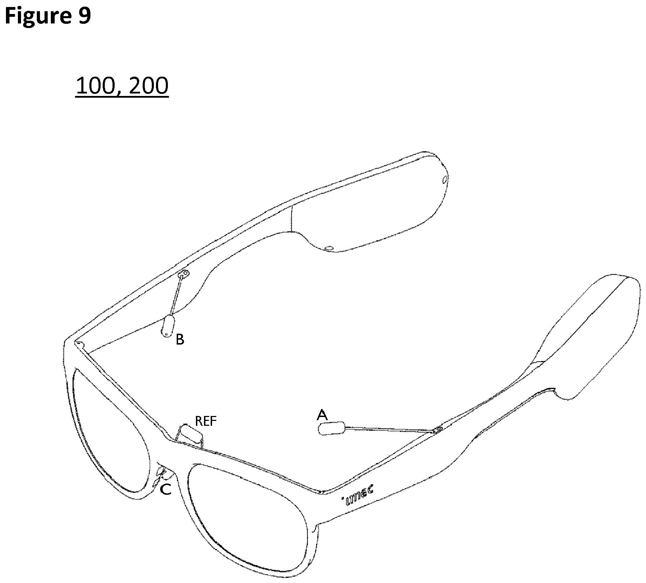

**Figure 9** shows a perspective view of an example wearable device **100, 200** for eye activity monitoring in the form factor of a pair of glasses. The wearable device **100, 200** comprises a plurality of EOG electrodes **103**, associated electrode circuitry **104** and may even comprise a data processing unit **106** as in **figures 6,7**. The wearable device **100, 200** may acquire, determine and provide eye activity information based on a two-electrode configuration (single diagonal electrode pair), a four-electrode configuration (two diagonal electrode pairs) and/or a five-electrode configuration (two diagonal electrode pairs plus a reference electrode).

Advantageously, one, two or three EOG electrodes (electrodes **C,D,REF** as shown in **Figure 10**) may be located in the region of the wearable structure touching the nose of the subject. Two additional electrodes **A,B** may be also linked to the wearable device and configured for touching or being placed in the facial area of the subject between temporal and frontalis muscles. It shall be noted that the wearable device **100, 200** of **figures 9, 10** is just an example embodiment and other implementation form factors and designs are possible. For example, according to an embodiment, the wearable device **100, 200** may be implemented in the form factor of AR, VR goggles or VR display devices and the EOG electrodes may be integrated in the foam mask of the wearable device.

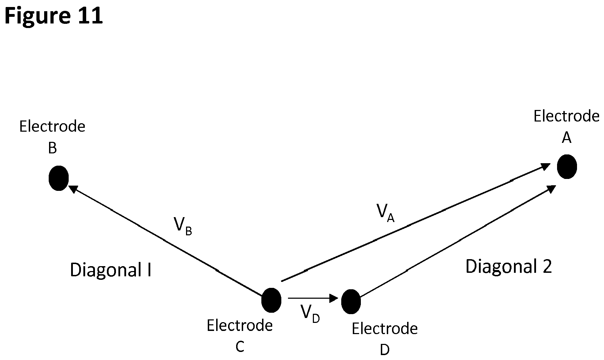

**Figure 11** illustrates a method for measuring EOG signals in a system for monitoring eye activity of a subject according to a first example embodiment, comprising four EOG electrodes **A,B,C,D** an in which one of the electrodes is a reference electrode. According to an embodiment, in order to measure the electrical potential on the electrodes in position **A,B,C,D**, one electrode is used as reference, and the EOG diagonals vectors are derived as combination of the other potentials:

$$VREF = VC$$

$$Diagonal\ 1 = VB$$

$$Diagonal\ 2 = VA - VD$$

**Figure 12** illustrates a method for measuring EOG signals in a system for eye activity monitoring according to a second example embodiment, comprising five EOG electrodes **A,B,C,D, REF** an in which one of the electrodes **REF** is a reference electrode. According to an embodiment, the reference electrode **REF** can be placed anywhere in the face of the user. According to example embodiments, the reference electrode **REF** may be placed in the forehead of the subject, or between electrodes **A, B**, or in the top of the nose area between the eyes. In this electrode configuration, the EOG diagonal vectors are derived as follows:

$$Diagonal\ 1 = VB - VC$$

$$Diagonal\ 2 = VA - VD$$

According to an example embodiment, the wearable device **100, 200** of **figures 9, 10** is implemented using a five-electrode configuration as described in **figure 12**, comprising: a reference electrode **REF** placed in the middle of the nose slightly above the eyes, a first electrode **A** placed at left temple area, a second electrode **B** placed at right temple area; a third electrode **C** placed at the right nose area, and a fourth electrode **D** placed at left nose area. The EOG activity is measured between the reference electrode **REF** and all the other electrodes. Thus, four eye signals are measured. The two diagonal vectors can be derived by combining these 4 signals by addition and subtraction.

It shall be noted that the reference electrode location can be changed to any other location on the face and the body of the user. However, according to embodiment of **figures 9, 10**, the reference electrode **REF** location is very well suited for good skin contact using the current form factor of the goggles, placed in the middle of the nose slightly above the eyes. Other form factors could change the reference electrode location. It is even possible to replace the reference electrode with one of the other electrodes (at temple or nose area) without losing functionality of the diagonals as described in this document. Therefore, in another embodiment, four electrodes could be used (1 at left temple area, 1 at right temple area, 1 at left nose area, 1 at right nose area), with 1 electrode being the reference electrode, and the diagonals being derived from the four activity measurements.

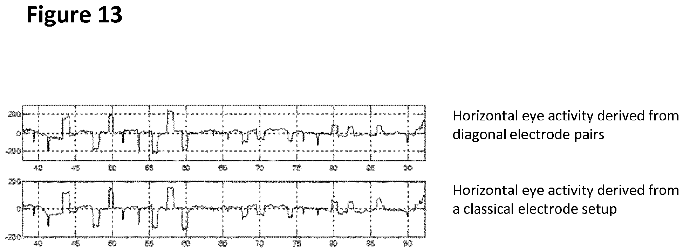

**Figure 13** shows a graph of the horizontal eye activity information derived from diagonal electrode pairs according to an example embodiment compared to a prior art electrode configuration. The top plot shows the horizontal eye activity from the determined diagonal channels according to the current description and the bottom plot the horizontal eye activity derived from a classical EOG electrode setup. The top plot shows the horizontal electrical eye activity measured after combining the two diagonal measurements. The Signal-to-Noise ratio of the diagonal configuration is larger than the SNR of the classical EOG electrode configuration setup.

It shall be noted that the system **1** system for monitoring eye activity of a subject according to embodiments herein described may comprise units and modules that may be implemented according to hardware and/or software state of the art techniques, comprising for example a microprocessor, microcontroller or digital signal processor that can understand and execute software program instructions. Some programmable hardware logic and memory means may be specifically designed also for executing the signal processing methods or parts of it according to exemplary embodiments. It is also understood that the units of the system described may be implemented in distributed locations, for example in a mobile device and/or a network. Any storage units needed may be implemented as a unique storage memory and/or a distributed memory.

**Claims**

1. An electronic system (1) for monitoring eye activity of a subject, comprising:

   a plurality of EOG electrodes (103,A,B,C,D,REF);
   electrode circuitry (104) configured for measuring an adapt EOG signals received from the plurality of electrodes (103,A,B,C,D,REF); and
   a data processing unit (106) configured for performing EOG signal processing and eye activity determination;
   wherein the data processing unit (106) is configured for performing eye activity determination based on at least a first diagonal eye biosignal vector (Diagonal 1) derived from the biosignals measured between at least a first electrode pair (B,C) that, in operation, is located on a first diagonal plane (D1) around a first subject's eye.

2. An electronic system (1) according to claim 1, wherein the first electrode pair (B,C) comprises a first electrode (B) located in the facial area of the subject between temporal and frontalis muscles and a second electrode (C) located on the nose below the eye of the subject.

3. An electronic system (1) according to any of the previous claims, wherein the diagonal plane (D1) around the subject's eye is neither parallel nor perpendicular to a horizontal plane (H) defined by the eyes of the subject.

4. An electronic system (1) for monitoring eye activity of a subject according to any of the previous claims, further configured for performing eye activity determination based also on a second diagonal eye biosignal vector (Diagonal 2) derived from the biosignals measured between a second electrode pair (A,D) that, in operation, is located on a second diagonal plane (D2) around a second subject's eye.

5. A wearable device (100,200) for monitoring eye activity of a subject, comprising:

   a plurality of EOG electrodes (103,A,B,C,D,REF) and electrode circuitry (104) configured for measuring an adapt EOG signals received from the plurality of electrodes;
   wherein, the plurality of EOG electrodes (103,A,B,C,D,REF) are configured in electrode measurement pairs, comprising at least a first electrode pair (B,C) that, when the wearable device is placed on the face of the subject, is located on a first diagonal plane (D1) around the subject's eye and the electrode circuitry (104) is configured for measuring the EOG signals between said first electrode pair (B,C).

6. A wearable device (100, 200) according to claim 5, wherein a first electrode pair (B,C) comprises a first electrode (B) configured for touching the facial area of the subject between temporal and frontalis muscles and a second electrode (C) configured for touching the nose below the eyes of the subject.

7. A wearable device (100, 200) according to claims 5 or 6 wherein the at least first electrode pair (B,C) is integrated in a pair of googles.

8. A method for monitoring eye activity of a subject, comprising:

receiving a plurality of EOG signals from a plurality of electrodes (103,A,B,C,D,REF), said EOG signals being measured between at least a first electrode pair (B,C) that, in operation, is located on a first diagonal plane (D1) around the subject's eye; and
performing EOG signal processing and eye activity determination based on at least a first diagonal eye biosignal vector (Diagonal 1) derived from said biosignals measured between said at least first electrode pair (B,C).

## Figure 1A

## Figure 1B

Electrode B
Above left/right eye

Electrode D
Right side of right eye

Electrode A
Left side of left/right eye

Electrode C
Beneath left/right eye

Horizontal eye
movement
information

Vertical eye
movement
information

## Figure 2

Electrode location
area above the eyes
50

Electrode location
area below the eyes
51

## Figure 3A

## Figure 3B

## Figure 4A

```
         ┌──────────────┐
         │    start     │
         └──────────────┘
                │
                ▼
      ┌──────────────────┐    400
      │      Obtain      │
      │ electrooculograms│
      └──────────────────┘
   ┌──────────│───────────────────────┐
   │          ▼                        │    106
   │  ┌──────────────────┐   401       │
   │  │Process and combine│            │
   │  │ electrooculograms │            │
   │  └──────────────────┘            │
   │          │                        │
   │          ▼                        │
   │  ┌──────────────────┐   402       │
   │  │ Detect eye activity│           │
   │  └──────────────────┘            │
   └──────────│───────────────────────┘
                ▼
         ┌──────────────┐
         │     end      │
         └──────────────┘
```

## Figure 4B

```
         ┌──────────────┐
         │    start     │
         └──────────────┘
                │
                ▼
      ┌──────────────────┐    400
      │      Obtain      │
      │ electrooculograms│
      └──────────────────┘
   ┌──────────│───────────────────────┐
   │          ▼              403       │    106
   │  ┌──────────────────┐            │
   │  │     Process      │            │
   │  │ electrooculograms │            │
   │  └──────────────────┘            │
   │          │                        │
   │          ▼              404       │
   │  ┌──────────────────┐            │
   │  │ Detect eye activity│           │
   │  └──────────────────┘            │
   └──────────│───────────────────────┘
                ▼
         ┌──────────────┐
         │     end      │
         └──────────────┘
```

**Figure 5**

```
                                    ┌──────────────┐
                                    │    start     │
                                    └──────┬───────┘
                                           │
                                           ▼
                                    ┌──────────────────┐
                                    │ Processed & combined │  ⟜ 500
                                    │ electrooculograms │
                                    └──────┬───────────┘
                                           │
        503                                ▼
         ⟜  ┌──────────────┐       ┌──────────────────┐
            │              │ ────► │ Eye movement and eye │  ⟜ 501
            │ Signal quality│ ◄──── │  blink detection  │
            │  estimator   │       └──────┬───────────┘
            │              │              │
            │              │ ────►        ▼
            │              │       ┌──────────────────┐
            └──────────────┘ ◄──── │   Interpreter    │  ⟜ 502
                                   └──────┬───────────┘
                                          │
                                          ▼
                                   ┌──────────────┐
                                   │     end      │
                                   └──────────────┘
```

## Figure 6

<u>1</u>

## Figure 7

<u>1</u>

# Figure 8

104

301

Acquisition

302

Electrodes
connection

303

Data handling and
processing

Electronics

**Figure 9**

<u>100, 200</u>

**Figure 10**

<u>100, 200</u>

**Figure 11**

**Figure 12**

# Figure 13

Horizontal eye activity derived from diagonal electrode pairs

Horizontal eye activity derived from a classical electrode setup

Europäisches
Patentamt

European
Patent Office

Office européen
des brevets

# EUROPEAN SEARCH REPORT

Application Number

EP 19 17 0306

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/150897 A1 (KOMAKI HIROAKI [JP]) 1 June 2017 (2017-06-01) * paragraphs [0045] - [0047], [0142], [0156] - [0163]; figures 2,19 * | 1-8 | INV. A61B5/0496 A61B5/00 A61B5/16 A61B3/113 |
| X | US 2013/242077 A1 (LIN CHIN-TENG [TW] ET AL) 19 September 2013 (2013-09-19) * paragraphs [0021] - [0029]; figures 2,3 * | 1-8 | |
| X | US 2018/341328 A1 (AGELL CARLOS [BE] ET AL) 29 November 2018 (2018-11-29) * paragraphs [0067] - [0074] * | 1-8 | |
| X | US 5 726 916 A (SMYTH CHRISTOPHER C [US]) 10 March 1998 (1998-03-10) * column 6, lines 4-20; figure 2 * | 1-8 | |
| A | US 2010/069775 A1 (MILGRAMM MICHAEL [US] ET AL) 18 March 2010 (2010-03-18) * paragraph [0125] * | 1-8 | |
| A | US 6 416 480 B1 (NENOV VALERIY [US]) 9 July 2002 (2002-07-09) * column 12, line 51 - column 13, line 6 * | 1-8 | TECHNICAL FIELDS SEARCHED (IPC) A61B |

The present search report has been drawn up for all claims

| Place of search | Date of completion of the search | Examiner |
|---|---|---|
| Berlin | 15 October 2019 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

           

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 17 0306

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-2019

| Patent document cited in search report | | Publication date | Patent family member(s) | | Publication date |
|---|---|---|---|---|---|
| US 2017150897 | A1 | 01-06-2017 | JP 6426589 B2<br>JP 2017093984 A<br>US 2017150897 A1 | | 21-11-2018<br>01-06-2017<br>01-06-2017 |
| US 2013242077 | A1 | 19-09-2013 | TW 201338749 A<br>US 2013242077 A1 | | 01-10-2013<br>19-09-2013 |
| US 2018341328 | A1 | 29-11-2018 | EP 3407164 A1<br>JP 2018196730 A<br>US 2018341328 A1 | | 28-11-2018<br>13-12-2018<br>29-11-2018 |
| US 5726916 | A | 10-03-1998 | NONE | | |
| US 2010069775 | A1 | 18-03-2010 | NONE | | |
| US 6416480 | B1 | 09-07-2002 | NONE | | |

EPO FORM P0459

For more details about this annex : see Official Journal of the European Patent Office, No. 12/82